# EUROPEAN PATENT APPLICATION

(11) **EP 2 266 972 A1**
(43) Date of publication of application: **29.12.2010**
(21) Application number: 09305540.8
(22) Date of filing: 12.06.2009
(51) Int. Cl.: C07D 401/12, A61K 31/444

(54) **New chemical molecules that inhibit the splicing mechanism for treating diseases resulting from splicing anomalies**

(71) Applicant: Splicos, 34000 Montpellier (FR); Université Montpellier 2 Sciences et Techniques, 34000 Montpellier (FR); CENTRE NATIONAL DE LA RECHERCHE SCIENTIFIQUE (CNRS), 75016 Paris (FR); Institut Curie, 75005 Paris (FR)
(72) Inventor: Mahuteau Betezer, Florence, 78470, SAINT REMY-LES-CHEVREUSE (FR); Tazi, Jamal, 34830, CLAPIERS (FR)
(74) Representative: Le Coupanec, Pascale A.M.P.

(57) **Abstract**

The present invention relates to a compound of the formula I; a pharmaceutical composition comprising at least one such compound; and the use of such compound in preparing a drug to treat, in a subject, a genetic disease resulting from at least one splicing anomaly.

## Description

The invention relates to novel compounds for the preparation of compositions useful for the treatment of diseases resulting from changes in splicing processes.

Certain indole derivative compounds such as ellipticine derivatives and aza-ellipticine derivatives are already known as intercalating molecules for correcting dysfunctions in gene expression, notably in DNA replication. They have been more specifically described for treating diseases such as cancer, leukemia or AIDS (see in particular patents FR 2 627 493, FR 2 645 861, FR 2 436 786).

Concerning current treatments for AIDS, the various approaches aimed at reducing viral load in patients infected by HIV utilize molecules intended to inhibit the enzymatic activity of viral reverse transcriptase or of the protease involved in virus protein maturation. Regarding reverse transcriptase inhibitors, these can be nucleosidic (NRTIs), non-nucleosidic (NNRTIs) or nucleotidic in nature. The purpose of using these compounds is to prevent a DNA copy of the retroviral genome from being produced and, consequently, from being integrated into the genome of the host cell. Protease inhibitors (PIs) interfere with the proper maturation of viral proteins and cause the production of incomplete particles with altered infectious capacities. There is another type of anti-retroviral compound used for its ability to prevent viruses from entering the cell. These entry inhibitors can be either peptides that interfere with the fusion of viral glycoproteins gp41 or gp120 with the membrane of CD4 cells or molecules that target HIV cellular co-receptors CCR5 and CXCR4. The absence of cellular proteins resembling HIV integrase has also been exploited to develop novel anti-HIV molecules that inhibit this enzymatic activity. Although a number of integrase inhibitors are in the clinical trial phase, no molecule is yet available on the market.

Theintracellular splicing process consists of eliminating introns in pre-messenger RNAs to produce mature messenger RNAs that can be used by the translation mechanism of the cell (SHARP, Cell, vol. 77, p. 805-815, 1994). In the case of alternative splicing, the same precursor can be the source of messenger RNAs coding for proteins with distinct functions (BLACK, Annu. Rev. Biochem. vol. 72, p. 291-336, 2003). The precise selection of 5' and 3' splicing sites is thus a mechanism that generates diversity and that can lead to the regulation of gene expression according to the type of tissue or during the development of an organism. The factors involved in this selection include a family of proteins called SR, characterized by the presence of one or two RNA recognition motifs (RRM) and a domain rich in arginine and serine residues called an RS domain (MANLEY & TACKE, Genes Dev., vol. 10, p. 1569-1579, 1996). By binding to short exon or intron sequences of the pre-mRNA, called ESE (exonic splicing enhancer) or ISE (intronic splicing enhancer), SR proteins are able to activate, in a dose-dependant manner, sub-optimal splicing sites and to enable the inclusion of exons (GRAVELEY, RNA, vol. 6, p. 1197-1211, 2000). The activity of an SR protein in alternative splicing is specific insofar as the inactivation of the corresponding gene is lethal (WANG et al., Mol. Cell, vol. 7, p. 331-342, 2001).

Sequencing of the human genome and analysis of EST (expressed sequence tag) banks has revealed that 65% of genes are expressed in the form of alternatively spliced variants (EWING & GREEN, Nat. Genet., vol. 25, p. 232-234, 2000; JOHNSON et al., Science, vol. 302, p. 2141-2144, 2003). This mechanism is thus a favored target of modifications that can affect the factors involved in regulating splicing and of mutations that affect the sequences necessary for this regulation. At present, it is estimated that roughly 50% of the point mutations responsible for genetic diseases induce aberrant splicing. These mutations can interfere with splicing by inactivating or creating splicing sites, but also by modifying or generating regulating elements such as splicing enhancers or splicing silencers in a particular gene (CARTEGNI et al., Nat. Rev. Genet., vol. 3, p. 285-298, 2002; TAZI et al., TIBS, vol. 40, p. 469-478, 2005).

The strategies currently developed to correct these splicing defects rest on the use of various types of molecules (TAZI *et al.,* cited above, 2005).

One strategy aimed at developing novel molecules to correct or eliminate abnormal splicing, for example, rests on the overexpression of proteins that interfere with this type of splicing (NISSIM-RAFINIA et al., Hum. Mol. Genet., vol. 9, p. 1771-1778, 2000; HOFINANN et al., Proc. Natl. Acad. Sci. U.S.A., vol. 97, p. 9618-9623, 2000).

Other strategies rest on the use of antisense oligonucleotides (SAZANI et al., Nat. Biotechnol., vol. 20, p. 1228-1233, 2002; SAZANI & KOLE, Prog. Mol. Subcell. Biol., vol. 31, p. 217-239, 2003) or of PNA (CARTEGNI et al., Nat. Struct. Biol., vol. 10, p. 120-125, 2003) enabling, respectively, the inhibition or activation of a splicing event.

Yet another strategy rests on the identification of compounds that influence the splicing efficiency of the pre-mRNA of interest (ANDREASSI et al., Hum. Mol. Genet., vol. 10, p. 2841-2849, 2001).

Lastly, a strategy based on the use of trans-splicing to replace mutant exons has been described (LIU et al., Nat. Biotechnol., vol. 20, p. 47-52, 2002).

One of the disadvantages of the developed strategies cited above to correct or eliminate abnormal splicing is their production cost. Indeed, the cost of producing antisense oligonucleotides that must be modified to improve their stability, and that of PNA molecules, is high.

Another disadvantage of the developed strategies cited above is that they require the use of expression vectors, such as, for example, for the strategy based on the use of trans-splicing.

International application WO05023255, under French priority of applications FR0310460 and FR0400973, filed by the Applicant, disclosed the use of indole derivatives to treat diseases related to the pre-messenger RNA splicing process in the cell.

Thus it was recently shown that certain indole derivatives prove particularly effective in treating metastatic cancer and in treating AIDS (BAKKOUR et al., PLoS Pathogens, vol. 3, p. 1530-1539, 2007).

However, the compounds described have a flat structure with four rings that have the disadvantage of intercalating between DNA bases and can thus lead to cellular toxicity.

In order to minimize the risk that these indole derivatives intercalate between DNA bases, the inventors developed novel compounds that are particularly effective in treating diseases related to the splicing process, but which, in a surprising manner, have a cellular toxicity that is clearly less than the indole derivatives of the prior art. In addition, these compounds are able to selectively inhibit certain splicing events.

A first object of the present invention relates to a compound of the following formula I : 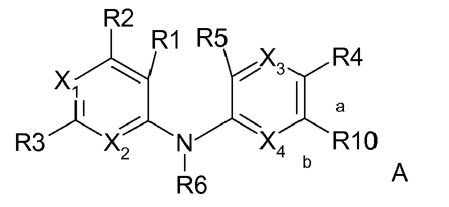 wherein :
X1, X2 and X3 independently represent a nitrogen atom, or a CR8 group, at least one of X1 and X2 being a nitrogen atom;
R8 represents a hydrogen atom or a halogen atom, a hydroxyl, alkyl, trifluoroalkyl, ester, ether, such as a methoxy or trifluoromethoxy group, or benzyl, optionally substituted, a nitro or a cyano group, preferably R8 represents a hydrogen atom,
when a ring A, defined below, is in position a, X4 represents a nitrogen atom or a CR8 group, and when a ring A is in position b, X4 represents a carbon atom part of the ring A,
R1, R2, R3 and R5 independently represent a hydrogen or a halogen atom, an alkyl, a trifluoroalkylgroup, ether, such as a methoxy or trifluoromethoxy group, or benzyl, optionally substituted, a nitro or a cyano group.
when the ring A is at position b, R4 represents a hydrogen atom, a halogen atom or an alkyl, a trifluoroalkyl, , ester, ether group, such as a methoxy or trifluoromethoxy group, or benzyl, optionally substituted, and when the ring A is at position a, R4 is a carbon atom part of the ring A,
R10 represents a carbon atom part of ring A,
R6 represents a hydrogen atom or an alkyl group,

A represents a ring at position a or b of formula I, said ring A corresponding to: 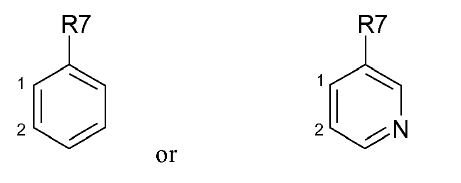 wherein :
R7 represents a hydrogen, or halogen atom or an alkyl, hydroxyl or amine group which can be linear or branched and/or unsaturated and optionally substituted,
pharmaceutically acceptable salts of said compounds, isomers thereof and/or mixtures of the same,
with the exception of the following compound: 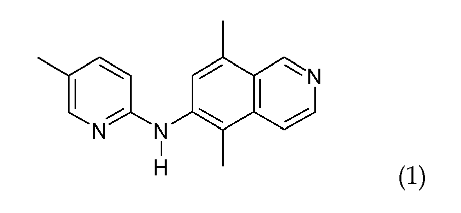 "Halogen atom" means the group comprising F, Cl, Br and I, preferably said halogen atom is a chlorine atom.
   "Unsaturated" means that the group comprises at least one double bond.

All the compounds disclosed in the examples are in the scope of the present invention.

Preferably, X1 represents a CR8 group when X2 represents a nitrogen group, and

X2 represents a CR8 group when X1 represents a nitrogen group.

Preferably, at least one of X3 and X4 is a nitrogen atom when the cycle A is in position a.

Preferably X3 and X4 are different, and even more preferably X3 represents a CR8 group when X2 represents a nitrogen group or a and X4 represents a CR8 group when X1 represents a nitrogen group.

Preferably, R1 represents a hydrogen atom or a methoxy group.

Preferably, R2, R3, R4 and R5 independently represent a hydrogen atom or a halogen atom or an alkyl, or benzyl, optionally substituted.

Preferably, R4 represents a hydrogen atom.

Preferably, R2 represents a hydrogen atom or a C1 to C4 alkyl group, preferably a methyl.

Preferably, R3, R5 and R6 independently represent a hydrogen atom.

Preferably, R7 represents a hydrogen, or halogen atom, more preferably a hydrogen or a chlorine atom.

Preferably, the ring A is attached at position a or b of the compound of formula I via the carbons numbered 1 and 2 in ring A.

Preferably, when the ring A is at position a, R4 is the carbon atom numbered 2 of the ring A, more preferably R4 is the carbon atom numbered 2 of the ring A and R10 is the carbon numbered 1.

Preferably, when a ring A is in position b, X4 is the carbon atom numbered 1 of the ring A, more preferably, X4 is the carbon atom numbered 1 of the ring A and R10 is the carbon numbered 2.

Preferably, the compound as described above does not include the following compounds: 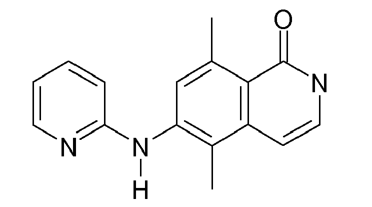 5,8-Dimethyl-6-(pyridin-2-ylamino)-2*H*-isoquinolin-1-one; 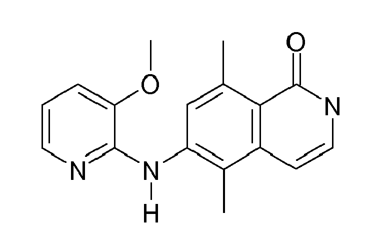 5, 8-diméthyl -6-(3 méthoxy-pyridin-2-ylamino)-isoquinolin-1-one 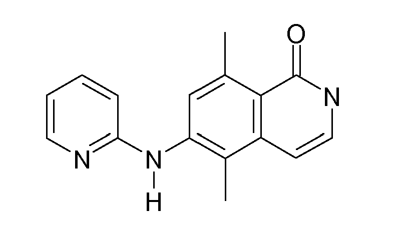 5,8-Dimethyl-6-(pyridin-2-ylamino)-2H-isoquinolin-1-one.

Advantageously, the compound of formula I is chosen among the group comprising: 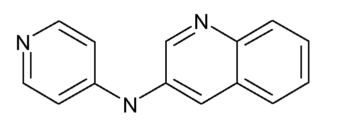 Pyridin-4-yl-quinolin-3-yl-amine; 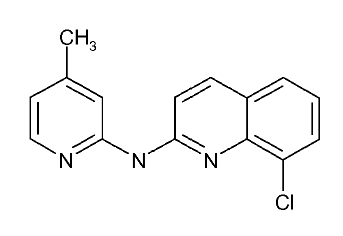 (8-Chloro-quinolin-2-yl)-(4-methyl-pyridin-2-yl)-amine; 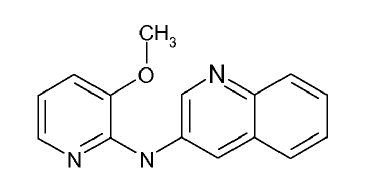 (3-Methoxy-pyridin-2-yl)-quinolin-3-yl-amine ; and 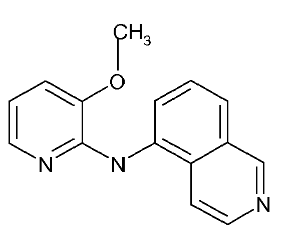 Isoquinolin-5-yl-(3-methoxy-pyridin-2-yl)-amine.

A second object of the invention consists of a pharmaceutical composition comprising at least one compound as described above and, optionally, a pharmaceutically acceptable support.

As examples of pharmaceutically acceptable supports, the composition can include emulsions, microemulsions, oil in water emulsions, anhydrous lipids and water in oil emulsions or other types of emulsions.

The inventive composition can further include one or more additives such as diluents, excipients, stabilizers and preservatives. Such additives are well known to those skilled in the art and are described notably in *"*Ullmann's Encyclopedia of Industrial Chemistry, 6th Ed." (various editors, 1989-1998, Marcel Dekker) and in "Pharmaceutical Dosage Forms and Drug Delivery Systems" (ANSEL et al., 1994, WILLIAMS & WILKINS).

A third object consists of the use of at least one compound of formula I in preparing a drug to treat, in a subject, a disease resulting from at least one splicing anomaly.

Therefore, the present invention relates to a compound of formula I for preparing a drug to treat, in a subject, a disease resulting from at least one splicing anomaly.

As used in the present application, the term "subject" refers to a mammal such as a rodent, cat, dog, primate or human, preferably said subject is a human.

Preferably, the inventive compounds have the ability to inhibit pre-messenger RNA splicing processes that are either constitutive or, more specifically, dependent on regulating sequences known as an ESE (exonic splicing enhancer), ISE (intronic splicing enhancer), ESS (exonic splicing silencer) and ISS (intronic splicing silencer).

In a particularly preferred way, splicing processes are either constitutive and/or or dependent on ESE regulating sequences.

Preferably, the present invention relates to the use of the at least one compound as described above for preparing a drug to treat, in a subject, AIDS.

Therefore, the present invention relates to a one compound as described above for treating AIDS.

A fourth object of the invention relates to a therapeutic method for treating a subject for a genetic disease resulting from splicing anomalies comprising the administration of a therapeutically effective quantity of a pharmaceutical composition as described above.

Preferably, said genetic disease resulting from splicing anomalies is AIDS.

A "therapeutically effective quantity" means a quantity that induces inhibition of the splicing of the pre-mRNAs of interest. Those skilled in the art will be able to determine said therapeutically effective quantity based on their general knowledge and on the methods described in the examples.

The compounds can be administered by any mode of administration such as, for example, by intramuscular, intravenous or oral route, etc.

In one embodiment according to the invention, said composition further includes an excipient making it possible to formulate the inventive compounds in such a way that said composition is provided in solid or liquid form to be prepared and administered by intravenous route.

The inventive compounds preferably will be administered by intravenous route at a concentration of 80-100 mg/m². The concentration will be chosen by those skilled in the art according to the organ or tissue to be treated, the state of advancement of the disease and the targeting mode used.

The following examples are provided as illustrations and in no way limit the scope of this invention.

### Example 1: Development of IDC16 derivative compounds

The inventors have shown that compound IDC16 (BAKKOUR *et al.,* cited above, 2007) interacts functionally with the SF2/ASF complex and thus contributes to blocking alternative splicing during HIV replication, leading to the termination of the production of Tat protein.

Accordingly, the family of polycyclic indoles, to which compound IDC16 belongs, is known to exhibit the properties of DNA intercalating agents. Such compounds thus present a risk in terms of undesirable side effects.

The inventors thus sought to develop novel molecules exhibiting activity comparable to IDC16, in terms of activity inhibiting HIV splicing, but while not exhibiting the characteristics of DNA intercalating agents.

In their initial hypothesis, the inventors considered that the two polar heterocycles at the two ends of compound IDC16 were associated with its activity and that the two median rings were of less importance.

Based on this hypothesis, the inventors considered that:
- the nitrogen of the indoline and of the D ring of IDC16 might act as acceptors of hydrogen bonds;
- the N-methylated 4-pyridinone motif might be preserved in the analogues;
- the flat tetracyclic geometry was not optimal and it might be wise to replace the B and C rings by other motifs to limit DNA intercalating properties.

### Example 2: Method for synthesizing the compounds of the present invention

### Typical procedure for Pd-catalysed aminations

To a solution of halogeno compound (0.5 mmol, 1 equiv) in tert-butanol (2 mL) were added the amino moiety (0.55 mmol, 1.1 equiv), Cs₂CO₃ (456 mg, 1.4 mmol, 2.8 equiv), Xantphos (4,5-Bis(diphenylphosphino)-9,9-dimethylxanthene ) (5.8 mg, 0.01 mmol, 2 mol %), Pd(OAc)₂ (2.2 mg, 0.01 mmol, 2 mol %). The reaction mixture was heated at 90 °C and stirred for 20 h under argon. The reaction mixture was concentrated under reduced pressure. The residue was purified by column chromatography on silica gel to yield pure compounds.

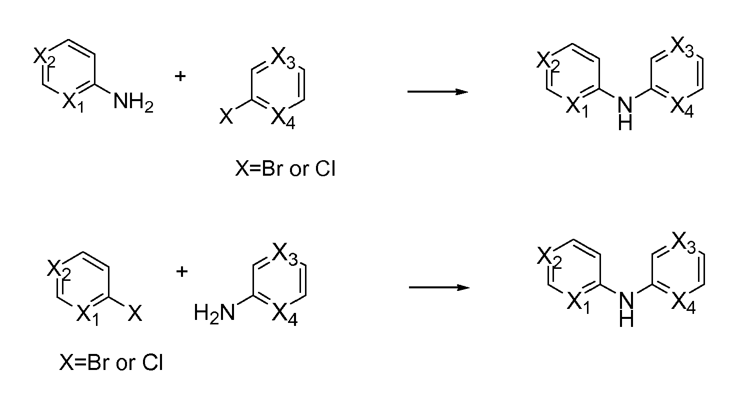

For example this procedure permitted to synthetize the following compounds:
Isoquinolin-5-yl-(3-methoxy-pyridin-2-yl)-amine
   ¹H NMR (300 MHz, CDCl3) δ 9.24 (s, 1H), 8.66 (dd, *J* = 1.7, 6.8, 1H), 8.55 (d, *J* = 6.0, 1H), 7.85 (d, *J* = 5.0, 1H), 7.76 (d, *J* = 6.0, 1H), 7.69 - 7.58 (m, 2H), 7.53 (s, 1H), 7.06 (d, *J* = 7.7, 1H), 6.78 (dd, *J* = 5.1, 7.8, 1H), 3.99 (s, 3H).
   ¹³C NMR (75 MHz, CDCl3) δ 153.23, 146.60, 142.97, 142.79, 138.53, 134.82, 129.53, 129.13, 127.95, 121.66, 119.82, 115.18, 115.05, 114.09, 100.15, 55.80.
(8-Chloro-quinolin-2-yl)-(4-methyl-pyridin-2-yl)-amine
   ¹H NMR (300 MHz, CDCl3) δ 8.82 (s, 1H), 8.17 (d, *J* = 5.1, 1H), 8.09 (s, 1H), 7.98 (d, *J* = 8.9, 1H), 7.76 (dd, *J* = 1.2, 7.6,1 1H), 7.61 (dd, *J* = 1.0, 8.0, 1H), 7.26 (t, *J* = 7.8, 2H), 7.15 (d, *J* = 8.7, 1H), 6.83 (d, *J* = 5.0, 1H), 2.46 (s, 3H).
   ¹³C NMR (75 MHz, CDCl3) δ 153.52, 153.14, 149.90, 147.43, 143.68, 138.08, 131.37, 129.98, 126.56, 125.58, 123.58, 119.17, 114.52, 114.02, 21.84.
(3-Methoxy-pyridin-2-yl)-quinolin-3-yl-amine
   ¹H NMR (300 MHz, DMSO) δ 9.17 (d, *J* = 2.5, 1H), 8.97 (d, *J* = 2.4, 1H), 8.79 (s, 1H), 7.94 - 7.79 (m, 3H), 7.58 - 7.46 (m, 2H), 7.31 (d, *J* = 7.9, 1H), 6.88 (dd, *J* = 5.0, 7.9, 1H), 3.94 (s, 3H).

### Example 3: Inhibition of HIV-1 production in infected peripheral blood mononuclear cells (PBMCs)

The first determination is that of the concentration of compound that exhibits the fewest side effects in terms of cell viability and progression of the cell cycle.

Within this framework, the peripheral blood mononuclear cells (PBMCs) of healthy donors are isolated by centrifugation on a FICOLL gradient. The cells are then cultivated to a density of 2.5 x 10⁶ cells/ml with RPMI medium supplemented with 1% inactivated human AB serum, then incubated at 37 °C, 5% CO₂ for an additional hour. The peripheral blood mononuclear cells are then recovered and cultivated for two days in RPMI medium supplemented with 10% fetal calf serum.

Part of the peripheral blood mononuclear cells (PBMC) is then cultivated for 72 hours in the presence of tritiated thymidine and phytohemagglutinin A (PHA) and in the presence or absence of the compounds of the present invention. Cell proliferation in the presence of the compounds of the present invention is finally measured by determining the incorporation of tritiated thymidine in the cellular DNA of the treated cells.

Another part of the peripheral blood mononuclear cells (PBMCs) that is activated (stimulated for 2 days with PHA and IL-2) is infected with HIV strains NL4.3 or Ada-M R5. The cells are then cultivated for 14 days in the presence of the compounds of the present invention. Viral replication is finally determined by quantifying protein p24 by the ELISA method. In parallel, cell viability is measured by exclusion with trypan blue in comparison with that of the untreated cells.

### • Example 4: Inhibition of HIV-1 production in infected macrophages

In order to generalize the HIV-1 replication effect of the molecules of the present invention to other cell types, we examined various steps of the viral cycle in cells treated with the various drug at a concentration of 5 µM and submitted to one-round infection.

For such experiences, macrophages can be infected by the Ada-M R5 HIV strain and treated for 18 hours with various concentrations of the compounds of the present invention. The culture medium is then eliminated and the cells washed with an abundance of PBS. The cells are then cultivated under normal conditions. The culture medium and the cells are then collected at days 4, 7 and 14. Finally, virus replication is measured indirectly by determining the level of p24 antigen in both the culture supernatant and the cellular lysate by the ELISA method. In parallel, cell viability of the macrophages in the presence of the compounds of of the present invention is measured as before.

For this purpose, we exposed HOS-CD4⁺-CCR5⁺ cells to defective virions obtained by cotransfecting 293T cells with a plasmid encoding the R5 envelope of the AD8 strain and another plasmid containing the entire HIV-1 genome mutated in the *envelope* gene and harbouring a *luciferase* marker gene fused to *nef* (Connor RI, Chen BK, Choe S, Landau NR. (1995) Vpr is required for efficient replication of human immunodeficiency virus type-1 in mononuclear phagocytes. Virology 206: 935-944.). The amounts of luciferase activity in cells infected with these virions reflect both the number of integrated proviruses and expression of multiply spliced species encoding nef/luc. Two days post-infection, luciferase activity in HOS-CD4+-CCR5+ infected cells was measured.

The results are shown below:

| Compound | Results |
|---|---|
| 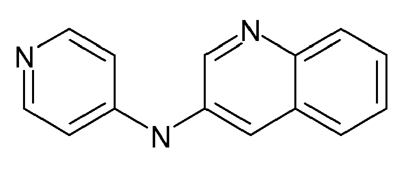 | + |
| 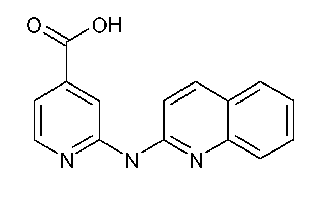 | - |
| 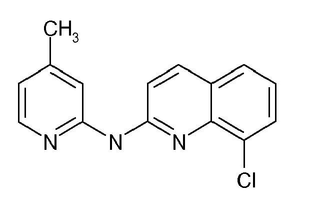 | + |
| 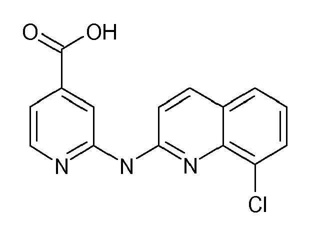 | - |
| 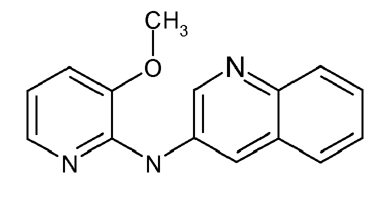 | + |
| 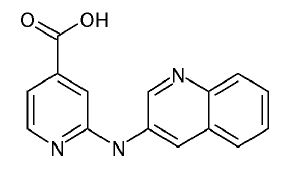 | - |
| 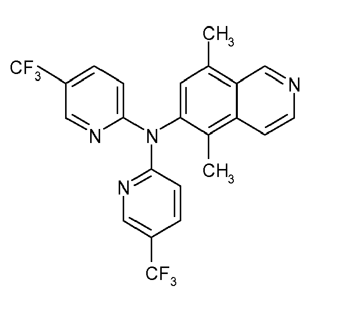 | - |
| 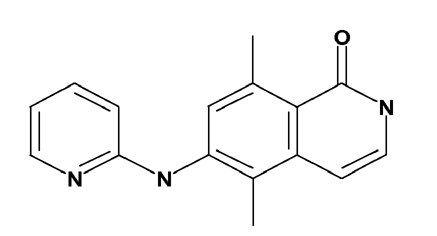 | - |
| 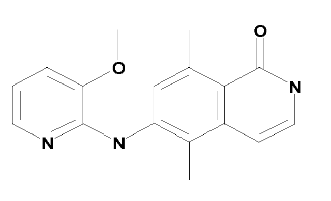 | - |
| 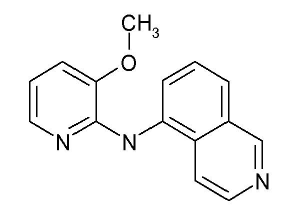 | + |
| 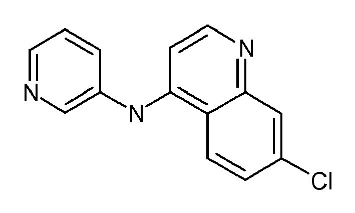 | - |

The results established that the compounds of the present invention show a luciferase inhibitory effect, thus showing that these compounds inhibit viral RNA splicing.

## Claims

1. Compound of the following formula I : 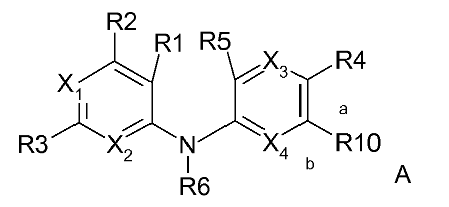 wherein :
X1, X2 and X3 independently represent a nitrogen atom, or a CR8 group, at least one of X1 and X2 being a nitrogen atom;
R8 represents a hydrogen atom or a halogen atom, a hydroxyl, alkyl, trifluoroalkyl, ester, ether, such as a methoxy or trifluoromethoxy group, or benzyl, optionally substituted, a nitro or a cyano group, preferably R8 represents a hydrogen atom,
when a ring A, defined below, is in position a, X4 represents a nitrogen atom or a CR8 group, and when a ring A is in position b, X4 represents a carbon atom part of the ring A,
R1, R2, R3 and R5 independently represent a hydrogen or a halogen atom, an alkyl, a trifluoroalkylgroup, ether, such as a methoxy or trifluoromethoxy group, or benzyl, optionally substituted, a nitro or a cyano group,
when the ring A is at position b, R4 represents a hydrogen atom, a halogen atom or an alkyl, a trifluoroalkyl, ester, ether group, such as a methoxy or trifluoromethoxy group, or benzyl, optionally substituted, and when the ring A is at position a, R4 is a carbon atom part of the ring A,
R10 represents a carbon atom part of ring A,
R6 represents a hydrogen atom or an alkyl group,
A represents a ring at position a or b of formula I, said ring A corresponding to: 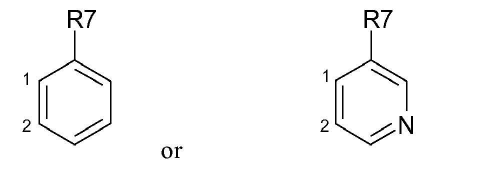 wherein :
R7 represents a hydrogen, or halogen atom or an alkyl, hydroxyl or amine group which can be linear or branched and/or unsaturated and optionally substituted,
pharmaceutically acceptable salts of said compounds, isomers thereof and/or mixtures of the same,
with the exception of the following compound:

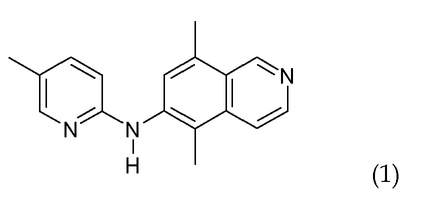

2. The compound of claim 1, wherein at least one of X3 and X4 is a nitrogen atom when the cycle A is in position a.

3. The compound of claim 1 or 2, wherein R1 represents a hydrogen atom or a methoxy group.

4. The compound of any of the previous claims, wherein R2 represents a hydrogen atom or a C1 to C4 alkyl group, preferably a methyl.

5. The compound of any of the previous claims, wherein R3, R5 and R6 independently represent a hydrogen atom.

6. The compound of any of the previous claims, wherein R7 represents a hydrogen or halogen atom, more preferably a hydrogen or a chlorine atom.

7. The compound of any of the previous claims, wherein the ring A is attached at position a or b of the compound of formula I via the carbons numbered 1 and 2 in ring A.

8. The compound according to claim 7, wherein the ring A is at position a.

9. The compound according to claim 8, wherein R4 is the carbon atom numbered 1 of the ring A.

10. The compound according to claim 7, wherein the ring A is in position b.

11. The compound according to claim 10, wherein X4 is the carbon atom numbered 2 of the ring A.

12. The compound of claim 1, **characterized in that** said compound is selected among the group comprising: 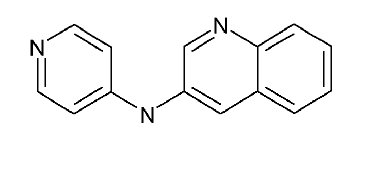 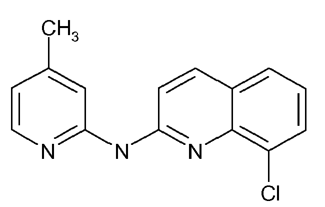 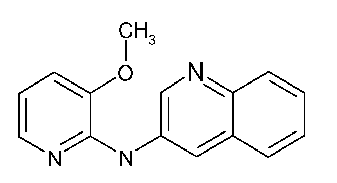 and

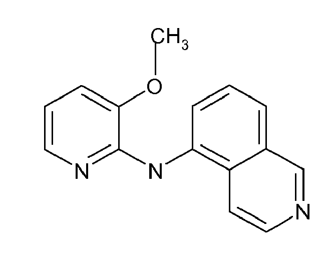

13. A pharmaceutical composition, **characterized in that** it comprises at least one compound of any of claims 1 to 12 and, optionally, a pharmaceutically acceptable support.

14. A compound according to any of claims 1 to 12
for preparing a drug to treat, in a subject, a disease resulting from at least one splicing anomaly.

15. The compound according to claim 14, **characterized in that** said disease is AIDS.
